(19) Europäisches Patentamt
European Patent Office
Office européen des brevets

(11) Publication number: **0 115 130**
**B1**

(12) **EUROPEAN PATENT SPECIFICATION**

(45) Date of publication of patent specification: **28.01.87**

(21) Application number: **83307304.2**

(22) Date of filing: **30.11.83**

(51) Int. Cl.[4]: **C 07 C 59/235,**
C 07 C 59/245, C 07 C 59/265,
A 61 K 31/28, A 61 K 31/30,
A 61 K 31/315

# (54) Pharmaceutical compositions for treating inflammation or arthritis.

(30) Priority: **06.12.82 US 447424**

(43) Date of publication of application:
**08.08.84 Bulletin 84/32**

(45) Publication of the grant of the patent:
**28.01.87 Bulletin 87/05**

(84) Designated Contracting States:
**AT BE CH DE FR GB IT LI NL SE**

(56) References cited:
**EP-A-0 046 409**
**GB-A- 827 521**
**GB-A-1 587 671**

(73) Proprietor: **NATIONAL RESEARCH LABORATORIES**
**3567 Blue Rock Road**
**Cincinnati, Ohio 45239 (US)**

(72) Inventor: **Maurer, Gerald L.**
**16 Woodsfield Court**
**Fairfield, OH (US)**

(74) Representative: **Thiemann, Peter Albert William et al**
**LLOYD WISE, TREGEAR & CO. Norman House**
**105-109 Strand**
**London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 115 130 B1**

**Description**

Inflammation is a local and protective response to tissue injury and destruction of cells. The precise elements constituting the inflammatory response vary according to the site of injury, the state of the body, and the injurious agent, such as bacteria or trauma. Should the inflammatory response become impaired or compromised, however, the corresponding tissue will undergo a degenerative process stimulating further injury and cell destruction. Obviously, then, the inflammatory response embodies a multifaceted process that is required to promote and rehabilitate normal tissue function. Therefore, since the inflammatory response is generally similar with various stimuli, it can be viewed and treated as a relatively non-specific response.

Inflammation may be manifested in numerous ways and one of the more well-known forms is arthritis. By definition, arthritis constitutes inflammation of a joint. Unfortunately, approximately 14% of the present United States population suffers from some type of arthritic manifestations. Further, if arthritis is ineffectively treated, it can develop into an extremely painful, degenerative and crippling disease. The present anti-arthritic therapy constitutes painful, toxic, inconvenient and ineffective protocols designed primarily to alleviate the symptoms rather than the causes. Thus, in light of current knowledge and the notorious effects of arthritis, there obviously is a critical need to develop an effective, safe, painless and convenient form of treatment that can be employed to alleviate successfully the causes arthritic inflammation.

In GB—A—1 587 671, a 1:1 metal complex of a multivalent heavy metal ion bound to a polyfunctional organic ligand is disclosed, effective as an anti-microbial agent and a metal transport agent.

In accordance with one aspect of the invention, the use of a metal complex of a multivalent heavy metal ion and a polyfunctional organic ligand in a ratio of 1:1 of the metal ion to the ligand, the ligand being either an organic acid or a substituted organic acid, the complex having an aqueous proton induced dissociation property represented by a sigmoidally-shaped curve on a cartesian coordinate plot of the negative log of the metal ion concentratiuon versus the negative log of the hydrogen ion concentration, characterised in that the use is for the manufacture of a medicament for alleviating an inflammatory disorder in an animal.

In accordance with another aspect of the invention, a composition containing a monometal complex of a multivalent heavy metal ion and a polyfunctional organic ligand in a ratio of 1:1 of the metal ion to the ligand, the ligand being either an organic acid or a substituted organic acid, the complex having an aqueous proton induced dissociation property represented by a sigmoidally-shaped curve on a cartesian coordinate plot of the negative log of the metal ion concentration versus the negative log of the hydrogen ion concentration characterised in that the composition has a pH which will not deleteriously dissociate the complex, and in that the composition is dispersed in a vehicle for either pharmaceutical anti-inflammatory topical application or subcutaneous administration.

Such concerns the treating of inflammation or arthritis by transporting metal ions in controlled amounts to the targeted inflammatory or arthritic areas. The particular class of metal complexes has been found especially suited to first deliver and then effectively release metal ions to prevent or reduce the inflammatory processes.

The 1:1 metal complex has an aqueous proton induced dissociation property represented by a sigmoidally-shaped curve on a cartesian coordinate plot of the negative log of the metal ion concentration versus the negative log of the hydrogen ion concentration. It has been found that metal complexes having such a dissociation property provide a means to transport copper, for instance, into the body and then enable its controlled release at the targeted inflammatory or arthritic sites.

Such thus fulfills a need in treating inflammation or arthritis where the intact transport of metal ions to the inflammatory or arthritic areas is required for controlled release of metal ion amounts upon demand. This is particularly illustrated in a topical anti-inflammatory or anti-arthritic application where metal ions are required to be introduced through the skin into the inflammatory or arthritic areas in large amounts. The skin is slightly acid, i.e., about pH 4.5 to 6.5 and the tissue, blood or stratum below the skin is closer to a pH of 7. Preferably, copper complexes are delivered through the skin intact for release of copper ions at the pathological pH of about 7 below the skin. Large amounts of metal ion are suitably released from the metal complexes at a pH of about 7, because of their relative instabilities at about pH 7 or the pathological pH below the skin. These metal complexes are very stable, even at high alkaline pHs, and relatively inert to organic moieties. Yet, upon demand, by reason of the unique dissociation property as demonstrated by sigmoidally shaped behavior on a pM—pH diagram, these agents offer controlled release of metal ions at a pH wherein inflammation or arthritis is believed to occur. Such method of transport employing a complex which dissociates upon demand at the pH most amenable to the activity of the moiety being acted upon, for example, the endogenous biochemical complexes that require copper for their activation to promote anti-inflammatory or anti-arthritic responses, is a unique and advantageous method to treat said diseases and causes of said diseases.

The anti-inflammatory or anti-arthritic metal complex has a dual character, i.e., aqueous solubility in high concentration by reason of its ionic character and a stability of the complexed metal. This property of solubility in water or neutral, acid, or alkaline media enables the production of concentrates capable of producing upon demand metal ions in the physiological range of about 4 to about 9, especially about 7.

The metal complex releases large amounts of metal ion from their coordinate structures most preferably at a pH of about 7 or less, i.e., where inflammatory or arthritic conditions are encountered. Upon demand, by reason of their unique dissociation property as demonstrated by sigmoidally shaped behavior on a pM—pH diagram, these anti-inflammatory or anti-arthritic agents offer controlled release of metal ions at a pH compatible with inflammatory or arthritic conditions.

The metal complex may be dispersed and buffered in a suitable vehicle to assist in its application. Such application preferably comprises topical or parenteral administration which includes topical application to the area of the skin where the inflammatory or arthritic disorder is believed to exist. In such vehicles, the neutralizing acidicity of the skin has been overcome so that the complexes will effecively release metal ions upon demand at the site of inflammation.

The invention will now be described by way of example, with reference to the following:—

The anti-inflammatory or anti-arthritic agent comprises a monometal complex of multivalent heavy metal and a polyfunctional organic ligand in a ratio of 1:1 of the metal to the ligand, the complex having a dissociation property represented by a sigmoidally shaped plot on a pM—pH diagram. Specific examples of the metal complex are dialkali metal monocopper (II) citrates represented by disodium-, dipotassium- or dilithium-monocopper (II) citrate. These dialkali monocopper (II) citrates have a dissociation property represented by a sigmoidal plot, wherein the curve of two directions meet at a point within the pH range of about 7 to about 9. It has been established that these monocopper (II) complexes in basic media, on the order of about pH 9 to about 12, are very stable, i.e., have an effective stability constant, $K_{eff}$, of the order of about $10^{12}$ to about $10^{13}$. However, $K_{eff}$ of these monocopper (II) citrate complexes at a pH of about 7—9 are on the order of about $10^{5}$ to about $10^{12}$. Therefore, at a pH of around 7, the effective stability constant of the monocopper (II) citrate complex is considerably lower (a thousand to several hundreds of thousand times lower) and a significant free $Cu^{++}$ concentration is available for anti-inflammatory and anti-arthritic activity. For example, about 10% of the copper in the complex is in the ionized state at or about pH 7 while approximately 0.1% of the copper is ionized at or about pH 9.

The anti-inflammatory or anti-arthritic complexes are consequently sensitive to pH, and as the pH is lowered to or below about 7, then copper ion is made more available. If tissue is intact, i.e., healthy without trauma, then there are few, if any, free endogenous reacting moieties to induce the dissociation of copper ions. If there is trauma caused by inflammation, then the copper ions are induced to dissociate and complex with the endogenous reacting moieties associated with such trauma, thereby reducing or alleviating the inflammation. In general, the complexes will then tend to dissociate over a pH range of about 3 to about 12. Above about pH 12, the complexes tend to be destroyed by the alkaline media, precipitating from the media as hydrous metal oxides. Below about pH 7, the instability of the metal complex results in high concentrations of the free $Cu^{++}$ upon demand, as explained to effect anti-inflammatory or anti-arthritic activities. At the pathological pH of about 7, below the skin, the controlled release is most effective. The complexes will preferably be dispersed in a vehicle to provide a composition having a pH of about 6.5 to about 9 for passage through the skin upon typical administration to provide controlled release of the metal ions upon presentment of endogenous reacting moieties that are associated with inflammatory or arthritic activities.

Other metal complexes of polyfunctional organic ligands exhibit the dissociation property characterized by a sigmoidal curve on a standard pM—pH diagram. For example, based upon the monometal-polyfunctional organic ligand complex, other metal ions of a monovalent of mulivalent nature, specifically, divalent and polyvalent cations including zinc, nickel, chromium, bismuth, mercury, silver, cobalt, and other similar metallic or heavy metal cations may be employed. Other polyfunctional organic ligands may be substituted for the citric acid already exemplified. Included among other polyfunctional ligands are the broader class of alpha or beta hydroxy polycarboxylic acids into which class the citric acid falls. Also, other functionally substituted acids such as alpha or beta amino, sulfhydro, phosphinol, etc., can be substituted in the molecular model of the metal complex and similar results can be achieved. In general, from a metal complex formula standpoint, the monometal complex of copper and citric acid corresponds to either of the following structural forms (A) and (B).

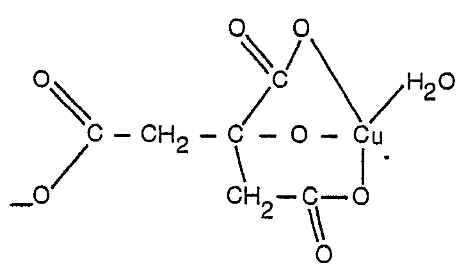

(A)

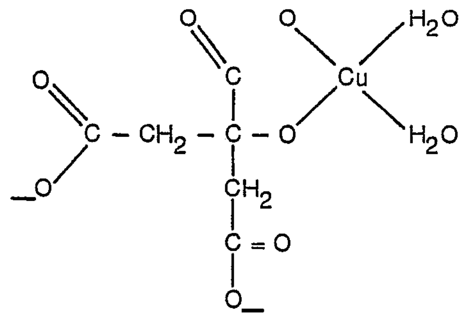

(B)

The (A) form is believed to be the preferred form by applying free energy considerations. A single proton introduced into the complex structure represented by either form (A) or (B) prevents deformation of stable five- or six-member coordinate rings. With the introduction of a proton, only seven-member rings may be formed by the coordination of the acetate electron donors and even such seven-member ring structures are unstable. Therefore, the complex molecule dissociates and presents the metal ion for its anti-inflammatory or anti-arthritic effects.

The (A) and (B) structural forms may be more generally represented by the following models:

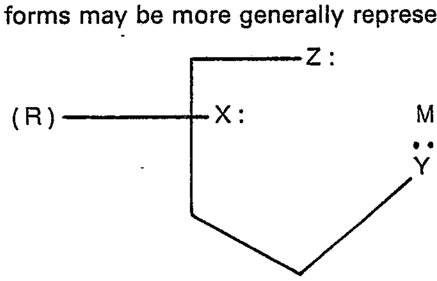

MODEL (A)

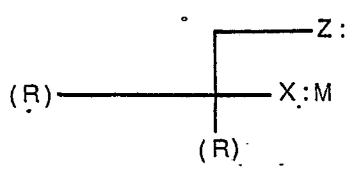

MODEL (B)

In the above models, the solid line segments represent a chemical bond between elements in the skeletal structure of the molecules; X, Y and Z represent electron pair donors; (R) represents any elemental or molecular species or group; M represents a metal and wherein the proton affinity of X is greater than that of Z, Y of R. It will therefore be appreciated that other Lewis base proton pairs, and other metal ions, may be substituted into these structural models for oxygen, divalent copper, or, for that matter, the carbon atoms to provide a molecular model which will similarly dissocate upon the introduction of one proton or similarly behaving species as exhibited by the sigmoidal behavior on a pM—pH diagram. The molecular models are thus alternative expressions for the metal complexes.

The unusual steric configuration of the molecules of these copper complexes imparts to it a dipolar nature which is characterized by the solvation of water at either pole in a rather rigid, highly polarized manner. This characteristic allows the hydrated complex to electrostatically adsorb to the surfaces of finely divided particles possessing either electronegative or electropositive surface characters. It also permits the "shielded" copper in the complex to migrate intact through membranes such as cell walls of microbes. While no present direct evidence of intact transport has been obtained, all collective results enable one to inductively reason that such transport occurs. $^{64}$Cu-citrate labelling should even quantitate the diffusion of 1:1 metal complexes through skin and cell membranes. The result is that the complex containing the metal ion can travel into areas which otherwise exclude similar compounds. For example, the application of copper salts to a proteinaceous membrane results in the attachment of the copper ions to the membrane components as in the formation of copper proteinates or salts. Little if any of the copper ion, in a soluble, ionized state progresses far beyond the membrane surface. Secondarily, copper salts tend to be corrosive in nature, causing various types of lytic reactions to occur with respect to animal tissue.

The proton induced dissociation of the copper complex renders copper ions to be made readily available as follows:

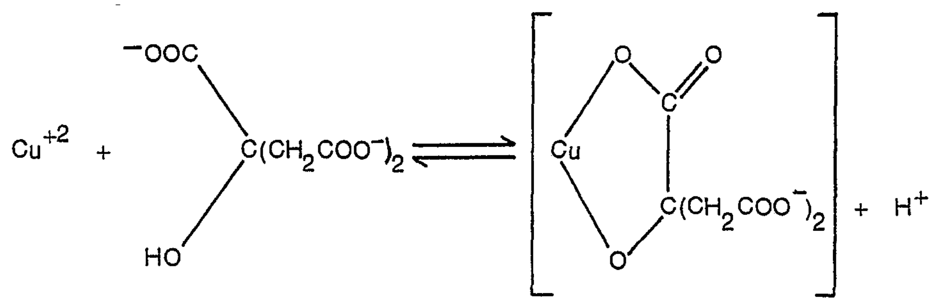

In addition, then, to the dipolar character of the 1:1 copper complex, it exhibits, most importantly, a relatively weak formation constant ($K_f$) meaning that the copper acceptors with stronger $K_f$ values can remove the copper ion from the 1:1 copper complex. This is accomplished in vivo by such structures as free amino acid groups, sulfhydryl radicals and any Lewis acid which can react with the coordination bonds holding the copper in place. That is, insertion of a hydrogen ion into the system can replace the displaced hydrogen ion originally associated with alcoholic hydroxyl group of the citrate moiety, thereby destabilizing the complex. Hence, the complex is characterized by possessing a proton induced dissociation character. This property is important in the pharmacodynamics of the copper complex in its use in the treatment of inflammation or arthritis.

In the topical application of the anti-inflammatory agents, it has been found that certain steps should be taken to obtain the desired results. The skin comprises a protective film covering the stratum corneum having a pH of about 4.5 to 6.5 attributed to the presence of free amino acid groups, sulfhydryl groups and other Lewis acids. Below the protective film, the remainder of the skin layers have a more neutral to basic pH of about 7.35 to about 7.45.

The anti-inflammatory compositions are buffered so as to neutralize the acidic environment initially encountered in the stratum corneum upon topical application, thereby enabling the copper complexes to pass through the skin for the desired activity below the skin upon demand. Such compositions having metal complex agents can thus traverse the acidic semi-permeable membrane of the skin intact and, once having traversed such membrane, release ionized copper species at the targeted inflammatory or arthritic sites.

The anti-inflammatory metal complexes are used to treat inflammation caused by injury, animal cell destruction or disease. Preferably, these metal compositions can be utilized to treat all forms of inflammation in animals including arthritis and more specifically osteoarthritis or rheumatoid arthritis. Further these metal complexes can be utilized to treat inflammation wherein the inflammation is for example synovitis, gonococcal arthritis, gout, spondylitis, or arthrosis deformans or any combination thereof. Still further, because of the anti-bacterial properties, these metal compositions can be utilized to treat infections associated with inflammation.

The anti-inflammatory metal complexes can be administered with topically or subcutaneously. The topical administration comprises physically applying and perfricating the anti-inflammatory metal complex to the epidermal area of the body affected with inflammation. The subcutaneous administration involves introducing the anti-inflammatory metal complexes directly below the skin at the targeted site, for example, therapeutic implants or inserts may be used to deliver the metal complexes.

The metal compositions diffuse through the skin and animal cell membranes intact. This is not to say that the metal complexes may not dissociate at all while diffusing through the skin or animal cell membranes. It is believed, however, that the efficiency of the complexes in use is attributed to the design of the metal complexes which maximizes the delivery of the metal ions to the targeted inflammatory or arthritic areas. In other words, a metal ion is transported through the skin and presented to inflammatory or arthritic areas having an endogenous reacting moiety demanding said metal ion wherein said metal ion is released in a controlled manner upon demand. Such provides the inflammatory area with greater amounts of metal ion for therapeutic treatment of inflammation. Further, the amount of metal ion released is controlled by the endogenous demanding moiety and pH at the inflammatory site. Still further, the unique properties of these metal complexes permit their incorporation into vehicles with an adjusted pH to minimize the amount of metal ions being released prior to reaching the targeted inflammatory area. In substance, these metal complexes are uniquely and advantageously designed to dissociate at physiological pH, maximizing the release of metal ions at the inflammatory sites having the desired demanding moieties.

As mentioned above, the metal complexes are dispersed in a suitable vehicle to form an embrocation that can be topically applied and perfricated. The main requirement for a suitable vehicle is a stable environment in which the metal complex can be dispersed to formulate a final embrocation that can be topically applied to the epidermis and perfricated. The vehicles can either be aqueous or oleophilic. Because of the dipole characteristic and stability of these metal complexes in aqueous environments, these novel metal complexes can be dispersed in oil and water dispersions. The vehicles that can be used are ointments and the more preferred forms are creams, gels, water or emulsion-like lotions. Anionic, nonionic and amphoteric emulsifying agents can be used to disperse the metal complexes in the vehicle. It has been

found that lotions are not as desirable for topical application as creams which provide a better consistency for perfrication. Further, creams and more specifically greaseless cold creams can be formulated to avoid or minimize the chemical incompatibilities which may otherwise occur between the metal complexes of this invention and the vehicles. Generally, the cold creams to be employed as vehicles comprise emulsifiers, hydrocarbon waxes, glycols and water. More particularly, the emulsifiers can be anionic, nonionic or amphoteric; the hydrocarbon waxes include petrolatum, wax, paraffin, ceresin, and synthetic polymer waxes, and the polyhydric alcohols or glycol ethers including diethylene glycol, propolyene glycol or glycerol. The emulsifiers are used to disperse the metal complexes in the cream, the hydrocarbon waxes are used as emollients and stiffening agents, while the glycols act as humectants to stabilize the emulsion. A preferred greaseless cold cream vehicle to be used with the metal complexes consists of glyceryl monostearate, white wax, ceresin, petrolatum (soft), glycerin and water. Typically, the anti-inflammatory composition comprises a metal complex dispersed in an oil-in-water emulsion with an emulsifying agent selected from the group of anionic, nonionic and ampyoteric agents by providing in the aqueous phase a dialkali metal monocopper (II) citrate in an amount of about 5% to about 15% w/w and more preferably about 10% w/w in a suitable vehicle at a pH of about 7 to about 9. A preferred anti-inflammatory composition comprises disodiummono-copper (II) citrate in an amount of about 10% w/w in a water-dispersible, greaseless cream base vehicle comprised of an oil-in-water emulsion having a buffered pH of about 7.

U.S. Patent Nos. 4,180,473, 4,129,509 and 4,055,655 disclose methods for the preparation of the 1:1 metal complexes, and methods for the determination of dissociation of such complexes. Such disclosures are incorporated herein by reference.

The invention will now be described in further detail with reference to the following which illustrates the preparation of the complexes, their activity and their use as anti-inflammatory or anti-arthritic agents:

## Preparation of the Metal Complexes and Anti-Inflammatory Compositions

The following methods were employed to prepare the anti-inflammatory compositions. Generally, the metal complexes were formed by complexing the metal ions with agents possessing reducing capabilities, such as reducing sugars, primary and secondary amines and structures with similar properties, to form complexes or salts. In the following examples, disodiummonocopper(II) citrate (MCC) was employed. This copper citrate complex was made as follows:

Ingredients:

65 ml water
61 gms citric acid, anhydrous
35 gms basic copper carbonate [$CuCO_3.Cu(OH)_2.H_2O$]
60 gms sodium bicarbonate ($NaHCO_3$)

The citric acid was dissolved in the water. The basic copper carbonate was added with stirring and dispersed well. This mixture was allowed to react for approximately 10 minutes or until the foam ($CO_2$ generation) subsided. Sodium bicarbonate was added slowly with gentle mixing until the pH was between 5.5 and 6.0. The solution was mixed until a black granular precipitate was no longer visible [$Cu(HCO_3)_2$]. The remainder of the sodium bicarbonate was added slowly with gentle stirring to adjust the pH to 7.0 for storage. The soluble copper chelate was thus prepared free from second salt.

Other techniques for making the complexes are set forth in U.S. Patent No. 4,278,610.

In order to formulate MCC into lotions, creams and the like, it was necessary to be very critical in the choice of ingredients. MCC was found to be chemically incompatible with many substances. As developed above, the anti-flammatory compositions must also provide a pH which will not deleteriously affect the activity of the complex. A unique and advantageous formulation of a skin cream base to be utilized with the metal complexes was found to satisfy the chemical demands and incompatibility problems of the metal complexes, e.g., MCC, as well as the aesthetic requirements as follows:

Greaseless Cold Cream Base

| | |
|---|---|
| Glyceryl monostearate | 11 gms |
| White wax | 2 gms |
| Ceresin | 3 gms |
| Petrolatum, soft | 4 gms |
| Glycerin | 6 gms |
| Water | 74 gms |
| Total | 100 gms |

The fatty acid ester, glyceryl monostearate, acts as an anionic emulsifier in the system to create an emulsion of "oil-in-water" and to disperse the metal complexes, e.g., MCC, into cream. Such an emulsifying agent has been found preferred. The white wax and ceresin acted as stiffening agents. The petrolatum (soft) was an emollient. Glycerin acted as a humectant to stabilize the emulsion. The cream base was made by simply weighing and combining all the ingredients in the order listed into a single container. The mixture, with slight and constant mixing, was brought to boiling, and then stirred until cooled. Violent agitation was avoided to eliminate beating into the cream too much air. If the dispersion was not satisfactory, the ingredients were reheated and then restirred until cooled.

In order to formulate an anti-inflammatory skin cream, powdered (spray-dried) MCC was added to the above skin greaseless cream base. The MCC employed was synthesized according to the above method which yielded a second salt-free product. The product of this synthesis was in an aqueous solution containing approximately 100 mg of copper $Cu^{++}$/ml. The spray-dried MCC was prepared by simple evaporation and, finally, in a Niro countercurrent 3048 stainless steel spray dryer which utilized wheel atomization and single point collection. The product obtained by this process was a finely divided blue powder of uniform particle size which contained approximately 3% moisture. The solid form of MCC was added to the desired percentage of w/w in various vehicles. That is, in order to manufacture a 10% w/w dispersion, a 10 gms portion of MCC spray-dried powder was added to 90 gms of the greaseless cream base prepared from the above-listed ingredients. The greaseless cream base was prepared as stated above. After cooling, a quantity of the base sufficient to make 100 gms was added to a vessel. The MCC powder was added with constant stirring which dissolved it in the water phase of the cream base. The level of active MCC was determined by extraction of the oil phase components employing normal butanol in saturated potassium chloride solution. To a tube containing equal volumes of the above was added a 1 gm aliquot of the MCC-greaseless cream base dispersion. The oil phase components were essentially totally extracted into the butanol while the water soluble constituents, including the MCC, were retained in the aqueous potassium chloride layer. Because of the high concentration of MCC, i.e., 10% w/w, direct spectrophotometric evaluation was performed at once to identify the complex by its absorption maximum of 738 nm., and its concentration was compared by its absorbance readings with those of standard solutions of MCC.

Other cream bases were employed which contained minimal amounts of substances known to react with the 1:1 complex copper, e.g., triethanolamine, disodium EDTA, stearic acid, and the like. If such a base was employed, more of the MCC than that calculated to comprise 10% w/w was incorporated so as to, in effect, satisfy the demand for copper by such endogenous demanding moieties. This approach was workable and has been employed in certain trials because the cream base, although containing certain relatively chemically incompatible substances, offered, for instance, improved texture, more pleasing coloration, and, was generally more available on a commercial scale than the above-described, essentially totally compatible, greaseless cream base formula.

Water Base

A simple MCC aqueous solution was prepared by dissolving a sufficient amount of powdered (spray-dried) MCC into distilled water, referred to hereinafter as "MCC liquid".

Lotion Base

A lotion formulation was prepared by introducing in a sufficient quantity powdered (spray-dried) MCC into a lotion vehicle comprising water, glycerin, mineral oil, stearic acid, glycol stearate and other ingredients, triethanolamine, acetylated lanolin alcohol, glyceryl stearate, acetyl alcohol, fragrance, dimethicone, magnesium aluminum silicate, methylparaben, propylene glycol, propylparaben, carbomer-934, disodium EDTA, D & C Red No. 19, D & C Yellow No. 10 and more commonly known as Vaseline Brand Intensive Care lotion. Again, as stated above, this lotion contained small amounts of EDTA which chelated, preferentially, some of the copper. Thus, a slight excess (less than 1% w/w) of powdered (spray-dried) MCC was added to correct this deficiency.

The above cream and lotion bases are adjusted to about 7 by employment of common acid, e.g., HCl, or base, e.g., NaOH. The systems are also buffered with sodium borate-boric acid and sodium carbonate-bicarbonate. Several types of the above preparations were retained for several years and exhibited satisfactory stabilities. Buffered creams or liquids prepared according to the above procedures, or similar procedures, were employed in the following examples.

Anti-Inflammatory and Anti-Arthritic Activity

The following are examples of a series of confirmed cases of osteoarthritis and rheumatoid arthritis treated with the topical metal complex. Generally, the medication was composed of the active ingredient, disodium-monocopper(II) citrate (MCC), C.A.S. Registry Number: 65330—59—8, dispersed in various vehicles including water, gels, creams and emulsion-type lotions. All of the cases incorporated herewith, as examples, were confirmed diagnostically by physicians employing the usual examining techniques, i.e., physical examination, radiography, etc. The ages of the patients who participated in these examples ranged from 38 years to 83 years. The average duration of illness in this population was 11 years, ranging from 3 to 25 years.

All of the patients in these examples prior to their treatment with the metal complexes had received standard courses of therapy including applications of heat; exercise; salicylates to tolerance; indomethacin; oral and intraarticular steroids. One of the patients had undergone total hip replacement prior to treatment. As least one of the patients had been treated previously with intravenous gold preparations and cytoxan. All of the patients had, through the course of the disease, grown progressively worse with reference to articular swelling and disturbances in articular mobility. Deformities, particularly in the interphalangeal articulations, continued to progress. A common, subjective symptom was arthralgia in all cases.

Upon instituting topical therapy with the copper complexes as disclosed herein, the patients were restricted from using any other medication including aspirin. Most patients had been using nothing but aspirin upon institution of the therapy, having given up on the usual remedies. Specific excerpts from each patient history are disclosed in the following examples. The topical anti-inflammatory or anti-arthritic embrocations employed in these examples were MCC liquids and/or MCC greaseless cold creams.

Example I

A 38 year old, male caucasian, Market Researcher, suffered from rheumatoid arthritis for a duration of 3 years. His symptoms were: pauciarticular, bilateral MCP's, no apparent systemic involvement.

Following treatment, flaring subsided within two weeks; increased range of motion with no pain by the third week; simultaneously, visible, measurable reduction in edema. After one month of daily application to the affected areas, treatment was stopped. Approximately three weeks later, flaring became evident. Treatment was begun again; flaring did not recur. The patient applied the medication once per week to the affected area and, both subjectively and objectively, was essentially asymptomatic. No adverse side effects of any kind were noted during treatment with these copper complexes. This patient relied heavily on typing as a part of his livelihood and was able to resume meaningful, productive activity.

Duration of treatment was 25 months. Dosage form: MCC Liquid, 10%; MCC Greaseless Cold Cream, 5% 10%.

Example II

An 83 year old, female caucasian, housewife, suffered from osteoarthritis for 25 years. Her symptoms were pauciarticular, bilateral PIPs, DIP.

Following treatment, pain diminished within one week; while motion was possible prior to treatment, it was extremely painful. After two weeks of treatment, tenting of the hands, which had been secondary to attempts to alleviate pain, was reduced so that the palmar surface of the hand was brought into approximately parallel contact with a flat surface. This patient complained of skin irritation at the sites of application. Examination of the patient revealed an extremely thin, onionskin-like stratum corneum. The concentration of the active ingredient was reduced approximately 10-fold; and the medication was applied at the same daily interval according to regimen. The irritation persisted and some pain was said to be felt to return. The original strength preparation was then employed at every-other-day intervals. The irritation subsided. The pain did not return. This patient was able to knit and crochet and perform various household tasks such as lifting and had, essentially, a vastly expanded range of motion compared to her pretreatment status. The patient continued to use the preparation on an every-other-day basis without experiencing any adverse side affects.

Duration of treatment was 23 months. Dosage form: MCC Greaseless Cold Cream, 1%, 10%

Example III

A 68 year old, male caucasian, tool and die maker, suffered from osteoarthritis for 10 years. His symptoms were pauciarticular, bilateral CMCs, PIPs.

The patient complained of extreme sensitivity to tactile stimuli and limited ability to grip tightly. Patient experienced flares on a weekly basis for approximately 10 years. Slight deformity in the PIPs of the index fingers was noted. Following treatment, flaring subsided within three weeks; the ability to grip tightly with no attendant pain was noted after three weeks. Following one month of daily application, the CMC and PIP edema was objectively reduced. Treatment of the right hand was stopped at this time; treatment of the left hand continued. After approximately three weeks, tenderness in the CMC and PIP of the index finger of the right hand returned; minimal flaring occurred according to the patient. A placebo preparation was applied to both hands, with cessation of treatment with active ingredient of the left hand also. Within two weeks, the patient complained of a return of the inability to grip tightly, stating that it was painful once again. The placebo was replaced with the active preparation and, within two days, the symtoms decreased. Within one week, the patient was asymptomatic. The patient has applied the product daily and has had no adverse side effects of any kind. This patient was required to exercise great manual dexterity in the performance of his job. He was able to return to work as a tool maker following treatment.

Duration of treatment was 22 months. Dosage form: MCC Greaseless Cold Cream, 0%, 1%, 2%, 5%, 10%.

Example IV

A 66 year old, male caucasian, master machinist, suffered from osteoarthritis for 7 years. His symptoms were pauciarticular, bilateral MCPs, PIPs, DIPs, and Right CMC.

This case is very similar to Example III, above. The third week of treatment was accompanied by a restored mobility in all affected joints with no attendant pain. His grip was strong and sure. He has continued to apply the medication daily and has had no sequellae. This patient had taken early retirement from his career as a master machinist due to his inability to hold tools; following one month of therapy, he returned on a part-time basis and has continued to work effectively with no arthritic manifestations.

Duration of treatment was 21 months. Dosage form: MCC Liquid, 5%, 10%; MCC Greaseless Cold Cream 5%, 10%.

Example V

A 65 year old, male caucasian, industrial engineer, suffered from osteoarthritis of the right knee for 9 years.

The medication was topically applied around the patellar margin on a twice-daily basis which resulted in a marked reduction of pain after ten days. The patient walked with a limp prior to treatment; two months after treatment was initiated, the patient walked with a normal gait. The patient continued to apply the medication on a daily basis and no adverse side effects were noted. The patient states that he was able to dress himself whereas he had required aid prior to treatment because of the stiff, painful knee condition.

Duration of treatment was 6 months. Dosage form: MCC Greaseless Cold Cream, 10%.

Example VI

A 60 year old, female caucasian, housewife, suffered from osteoarthritis for 3 years. Her symptoms were limited to the PIP and DIP of her right index finger.

Treatment was applied twice a day; after one week, patient stated that the pain was much less intense. Patient stated that she had no real limitation in motion but that the pain was constant and annoying but not unbearable. Three weeks of treatment resulted in the complete cessation of pain which did not return.

Duration of treatment was 6 months. Dosage form: MCC Greaseless Cold Cream, 10%.

Example VII

A 79 year old, male caucasian, retired banker, suffered from osteoarthritis for 9 years of the right shoulder. He had extremely limited range of motion without pain and was unable to lift his arm above his head without severe pain.

The medication was applied to the humero-clavicular joint area on a twice-daily basis. The pain subsided within two weeks and the patient was able to put on his hat with his right hand for the first time in about six years. Patient did continue to apply the preparation with no adverse side effects.

Duration of treatment was 13 months. Dosage form: MCC Greaseless Cold Cream, 10%.

Example VIII

A 52 year old, male caucasian, physician, suffered from rheumatoid arthritis for four years. His symptoms were pauciarticular of both hands and both wrists. His right hand manifestations were more pronounced and the area most severely affected was the MCP joint.

His range of motion was somewhat impaired and the pain was dull and constant. The application of the preparation resulted in an immediate tingling sensation in the area of application which persisted on a constant basis. He also reported having a metallic taste in his mouth about one hour following the application. The metallic taste, however, seemed to last for only a few minutes. The pain ceased approximately two weeks following initiation of treatment which consisted of twice-a-day application to the affected joints. The patient found that he had more freedom of motion of the more seriously affected MCP joints stating that he did not realize that he had been that "impaired". This patient stopped using the preparation and the MCP pain returned within one month. He reinstituted the therapy and continued to be asymptomatic with no adverse side effects.

Duration of treatment was 10 months. Dosage form: MCC Greaseless Cold Cream, 10%.

Example IX

A 68 year old, male causasian, dry cleaner/tailor, suffered from osteoarthritis for 16 years. His symptoms consisted of fusion of PIPs and MCPs on his left hand. Further, there was severe impairment of motion and complete inability to grasp objects with his right hand because of the apparent fusion of PIP and MCP on his right index finger.

Patient stated that he was unable to lift 5-gallon containers of cleaning fluid due to intense pain upon application of force to right hand. Patient applied the medication twice a day to the painful but not totally immobile PIP and DIP joints of the right hand. After one month, the pain had disappeared, as had edema which was palpable prior to the treatment. An approximate 4 mm decrease in the circumferential measurement of the involved PIP joints of the right hand indicated a definite recession of the inflammatory process. Patient stated that he is now able to lift heavy objects with no pain and was able to operate certain machinery in his business which he was unable to do for at least 10 years.

Duration of treatment was 10 months. Dosage form: MCC Greaseless Cold Cream, 10%.

Example X

A 72 year old, male caucasian, retired military officer, suffered from osteoarthritis for 10 years had monoarticular involvement in the right knee and was unable to walk without the aid of a cane.

His leg was stiff, due to incipient tetany secondary to self-imposed restriction of flexion in his attempt to minimize the pain. Radiographs indicated a severe inflammatory process with slight erosion of the femoral epiphysis. Spur formation, however, was not visualized. Medication was applied around the patellar margin on a twice-daily basis. In two weeks, the patient was able to flex the knee approximately 45° with no pain. After six weeks of application, the patient was able to both rotate and flex the knee in a full range. The patient continued to apply the medication on a twice-daily basis with no adverse side effects.

The patient was able to walk without a cane and was able to ambulate freely. He was involved in a course of physical therapy in an attempt to restore his muscle tone. He walked with a slight limp, probably due to the degenerative process noted above.

Duration of treatment was 11 months. Dosage form: MCC Greaseless Cold Cream, 10%.

Example XI

A 62 year old, male caucasian, retired steel mill worker, suffered from osteoarthritis of the right hip joint for 20 years.

His left hip had been surgically replaced with a complete prosthesis due to severe necrosis of the femoral articulation surfaces. Pain and lack of motion with minimal degeneration of the acetabulum and the femoral head had cause this patient to consider a second prosthesis. The medication was applied twice daily to the entire hip region from the iliac crest in a triangular fashion to approximately 5 cm below the articulation. After approximately one month, the pain upon motion was greatly reduced. After approximately six weeks, the pain was alleviated. A relatively full range of motion was restored to a point matching that of the prosthesis of the other hip. Radiography indicated no further degenerative change.

It would appear that surgical intervention was obviated in this patient. The patient continued to function in a normal fashion and used the medication once a day.

Duration of treatment was 19 months. Dosage form: MCC Greaseless Cold Cream, 10%.

Example XII

A 75 year old, female caucasian, retired clerk typist, suffered from osteoarthritis for 19 years. She had bilateral involvement of all DIPs, PIPs and MCPs with gross deformity but minimal fusion.

This patient had telescoping of the PIP of the left index finger. Her motion was severely limited and her ability to grasp objects was essentially absent. She had to be waited on by family members. After two weeks of therapy, which consisted of twice-a-day application of the medication, pain in all affected joints was markedly reduced. In the third week of treatment, range of motion of all joints except those of the right index finger was restored to approximately 50% level. One month following inception of treatment brought essentially complete relief from pain. Patient was referred to physical therapy and had approximately 75% restoration of function of both hands.

Dosage form: MCC Greaseless Cold Cream, 10%.

**Claims**

1. The use of a metal complex of a multivalent heavy metal ion and a polyfunctional organic ligand in a ratio of 1:1 of the metal ion to the ligand, the ligand being either an organic acid or a substituted organic acid, the complex having an aqueous proton induced dissociation property represented by a sigmoidally-shaped curve on a cartesian coordinate plot of the negative log of the metal ion concentration versus the negative log of the hydrogen ion concentration, characterised in that the use is for the manufacture of a medicament for alleviating an inflammatory disorder in an animal.

2. A use as claimed in Claim 1 wherein the complex is an aqueous admixture.

3. A use as claimed in Claim 2 wherein the admixture is oleophilic.

4. A use as claimed in either Claims 2 or 3 wherein the admixture is an oil and water dispersion.

5. A use as claimed in any one of Claims 2 to 4 wherein the admixture further contains a vehicle for the complex.

6. A use as claimed in Claim 5 wherein the vehicle comprises an emulsifier, a hydrocarbon wax, a polyhydric alcohol and water.

7. A use as claimed in either Claim 5 or 6 wherein the vehicle is a cream base comprising in an amount of about 11% w/w glyceryl monostearate, about 2% w/w white wax, about 3% w/w ceresin, about 4% w/w petrolatum, about 6% w/w glycerin, and about 74% w/w water.

8. A use as claimed in any preceding Claim wherein the complex is dispersed in a vehicle to provide a composition having a pH of either about 6.5 to about 9, about 7 to about 9, or above about 7.

9. A use as claimed in any preceding Claim wherein the metal ion is either copper, zinc, nickel, chromium, bismuth, mercury, silver or cobalt.

10. A use as claimed in any preceding Claim wherein the substituted organic acid is either hydroxy polycarboxylic, amino polycarboxylic, sulfhydro polycarboxylic, or phosphinol polycarboxylic.

11. A use as claimed in any one of Claims 1 to 8 wherein the metal complex is a dialkalimetal monoheavymetal chelate of an alpha-hydroxy polycarboxylic acid.

12. A use as claimed in Claim 11 wherein the complex is dialkalimetal monocopper (II) citrate.

13. A use as claimed in Claim 12 wherein the complex is disodium monocopper (II) citrate.

14. A composition containing a monometal complex of a multivalent heavy metal ion and a polyfunctional organic ligand in a ratio of 1:1 of the metal ion to the ligand, the ligand being either an organic acid or a substituted organic acid, the complex having an aqueous proton induced dissociation property represented by a sigmoidally-shaped curve on a cartesian coordinate plot of the negative log of the metal ion concentration versus the negative log of the hydrogen ion concentration characterised in that the composition has a pH which will not deleteriously dissociate the complex, and in that the composition is dispersed in a vehicle for either pharmaceutical anti-inflammatory topical application or subcutaneous administration.

15. A composition as claimed in Claim 14 wherein a composition has a pH in excess of about 7.

16. A composition as claimed in either Claim 14 or 15 wherein the complex is dispersed in a vehicle having a pH within the physiological range of about 7 to about 9.

17. A composition as claimed in any one of Claims 14 to 16 wherein the metal ion is either copper, zinc, nickel, chromium, bismuth, mercury, silver, or cobalt.

18. A composition as claimed in any one of Claims 14 to 17 wherein the substituted organic acid is either hydroxy polycarboxylic, amino polycarboxylic, sulfhydro polycarboxylic, or phosphinol polycarboxylic.

19. A composition as claimed in any one of Claims 14 to 16 wherein the metal complex is a dialkalimetal monoheavymetal chelate of an alpha-hydroxy polycarboxylic acid.

20. A composition as claimed in any one of Claims 14 to 16 wherein the complex is dialkalimetal monocopper (II) citrate.

21. A composition as claimed in any one of Claims 14 to 20 wherein the complex is an aqueous admixture.

22. A composition as claimed in Claim 21 wherein the admixture is oleophilic.

23. A composition as claimed in any one of Claims 14 to 22 wherein the vehicle comprises an emulsifier, a hydrocarbon wax, polyhydric alcohol and water.

24. A composition as claimed in Claim 23 wherein the vehicle is a cream base comprising glyceryl monostearate as the emulsifier.

25. A composition as claimed in any one of Claims 14 to 24 wherein the vehicle comprises glyceryl monostearate, white wax, ceresin, petrolatum, glycerin and water.

26. A composition as claimed in any one of Claims 14 to 16 wherein the metal complex is contained in a dispersion of oil and water with an emulsifier agent selected from anionic and non-anionic agents by providing in the aqueous phase a dialkalimetal monocopper (II) citrate in an effective anti-inflammatory amount at a pH of about 7 to about 9.

27. A composition as claimed in Claim 26 wherein the dialkalimetal monocopper (II) citrate is about 5% to about 15% w/w of the composition.

28. A composition as claimed in Claims 26 to 27 wherein the composition comprises disodium monocopper (II) citrate in an amount of about 10% w/w in a water-dispersible cream base vehicle comprising an oil-in-water emulsion having a pH of about 7.0.

29. A composition as claimed in any one of Claims 14 to 28 wherein the vehicle is either a cream base, emulsion, lotion or buffered solution.

30. A composition as claimed in any one of Claims 14 to 29 wherein the composition is in the form of a therapeutic implant for subcutaneous administration.

31. A composition as claimed in any one of Claims 14 to 29 wherein the composition is prepared for subcutaneous administration by injection.

**Patentansprüche**

1. Verwendung eines Metallkomplexes eines mehrwertigen Schwermetallions und eines polyfunktionellen organischen Liganden im Verhältnis 1:1 des Metallions zum Liganden, wobei der Ligand entweder einen organische Säure oder eine substituierte organische Säure ist, und wobei der Verlauf der wässrigen protoneninduzierten Dissoziation des Komplexes beim Auftragen des negativen Logarithmus der Metallionenkonzentration gegen den negativen Logarithmus der Wasserstoffionenkonzentration im kartesischen Koordinatensystem durch eine S-förmige Kurve darstellbar ist, dadurch gekennzeichnet, dass die Verwendung zur Herstellung eines Medikaments zur Linderung einer entzündlichen Tiererkrankung erfolgt.

2. Verwendung nach Anspruch 1, worin der Komplex eine wässrige Beimischung ist.

3. Verwendung nach Anspruch 2, worin die Beimischung oleophil ist.

4. Verwendung nach Anspruch 2 oder 3, worin die Beimischung eine Öl-und-Wasserdispersion ist.

5. Verwendung nach einem der Ansprüche 2 bis 4, worin die Beimischung ferner einen Träger für den Komplex enthält.

6. Verwendung nach Anspruch 5, worin der Träger aus einem Emulgator, einem Kohlenwasserstoffwachs, einem mehrwertigen Alkohol und Wasser besteht.

7. Verwendung nach Anspruch 5 oder 6, worin der Träger eine Cremegrundlage darstellt, die zu etwa 11% (Gew./Gew.) aus Glycerylmonostearat, etwa 2% (Gew./Gew.) weissem Wachs, etwa 3% (Gew./Gew.) Zeresin, etwa 4% (Gew./Gew.) Rohvaseline, etwa 6% (Gew./Gew.) Glycerin und etwa 74% (Gew./Gew.) Wasser besteht.

8. Verwendung nach einem der vorhergehenden Ansprüche, worin der Komplex in einem Träger dispergiert ist und dabei eine Zusammenseztung mit einem pH von entweder etwa 6,5 bis etwa 9, etwa 7 bis etwa 9, oder über etwa 7 ergibt.

9. Verwendung nach einem der vorhergehenden Ansprüche, worin als Metallion entweder Kupfer, Zink, Nickel, Chrom, Wismut, Quecksilber, Silber oder Kobalt vorliegt.

10. Verwendung nach einem der vorhergehenden Ansprüche, worin als substituierte organische Säure entweder eine Hydroxypolycarbonsäure, Aminopolycarbonsäure, Sulfhydropolycarbonsäure oder Phosphinolpolycarbonsäure vorliegt.

11. Verwendung nach einem der Ansprüche 1 bis 8, worin der Metallkomplex ein Dialkalimetall-Monoschwermetallchelat einer α-Hydroxypolycarbonsäure ist.

12. Verwendung nach Anspruch 11, worin der Komplex ein Dialkalimetall-Monokupfer(II)-citrat ist.

13. Verwendung nach Anspruch 12, worin der Komplex Dinatrium-Monokupfer(II)-citrat ist.

14. Zusammensetzung, enthaltend einen Monometallkomplex eines mehrwertigen Schwermetallions und eines polyfunktionellen organischen Liganden im Verhältnis 1:1 des Metallions zum Liganden, wobei der Ligand entweder eine organische Säure oder eine substituierte organische Säure ist, und wobei der Verlauf der wässrigen protonen-induzierten Dissoziation des Komplexes beim Auftragen des negativen Logarithmus der Metallionenkonzentration gegen den negativen Logarithmus der Wasserstoffionenkonzentration im kartesischen Koordinatensystem durch eine S-förmige Kurve darstellbar ist, dadurch gekennzeichnet, dass die Zusammensetzung einen pH aufweist, bei dem der Komplex nicht nachteilig dissoziiert, und dass die Zusammensetzung in einer Träger zur pharmazeutischen entzündungshemmenden äusserlichen Anwendung oder subkutanen Verabreichung dispergiert ist.

15. Zusammensetzung nach Anspruch 14, bei welcher der pH über etwa 7 liegt.

16. Zusammensetzung nach Anspruch 14 oder 15, worin der Komplex in einem Träger mit einem pH innerhalb des physiologischen Bereichs von etwa 7 bis etwa 9 dispergiert ist.

17. Zusammensetzung nach einem der Ansprüche 14 bis 16, worin als Metallion entweder Kupfer, Zink, Nickel, Chrom, Wismut, Quecksilber, Silber oder Kobalt vorliegt.

18. Zusammensetzung nach einem der Ansprüche 14 bis 17, worin als substituierte organische Säure entweder eine Hydroxypolycarbonsäure, Aminopolycarbonsäure, Sulfhydropolycarbonsäure oder Phosphinolpolycarbonsäure vorliegt.

19. Zusammensetzung nach einem der Ansprüche 14 bis 16, worin der Metallkomplex ein Dialkalimetall-Monoschwermetallchelat einer α-Hydroxypolycarbonsäure ist.

20. Zusammensetzung nach einem der Ansprüche 14 bis 16, worin der Komplex Dialkalimetall-Monokupfer(II)-citrat ist.

21. Zusammensetzung nach einem der Ansprüche 14 bis 20, worin der Komplex eine wässrige Beimischung darstellt.

22. Zusammensetzung nach Anspruch 21, worin die Beimischung oleophil ist.

23. Zusammensetzung nach einem der Ansprüche 14 bis 22, worin der Träger aus einem Emulgator, einem Kohlenwasserstoffwachs, einem mehrwertigen Alkohol und Wasser besteht.

24. Zusammensetzung nach Anspruch 23, worin der Träger eine Cremegrundlage mit Glycerylmonostearat als Emulgator ist.

25. Zusammensetzung nach einem der Ansprüche 14 bis 24, worin der Träger aus Glycerylmonostearat, weissem Wachs, Zeresin, Rohvaseline, Glycerin und Wasser besteht.

26. Zusammensetzung nach einem der Ansprüche 14 bis 16, worin der Metallkomplex in einer Dispersion von Öl und Wasser mit einem unter anionischen und nicht-ionischen Mitteln ausgewählten Emulgator enthalten ist, wobei in der wässrigen Phase ein Dialkalimetall-Monokupfer(II)-citrat in einer wirksamen entzündungshemmenden Menge bei einem pH von etwa 7 bis etwa 9 vorliegt.

27. Zusammensetzung nach Anspruch 26, worin das Dialkalimetall-Monokupfer(II)-citrat etwa 5% bis etwa 15% (Gew./Gew.) der Zusammensetzung ausmacht.

28. Zusammensetzung nach Anspruch 26 oder 27, worin die Zusammensetzung Dinatrium-Monokupfer(II)-citrat in einer Menge von etwa 10% (Gew./Gew.) in eiem in Wasser dispergierbaren Cremegrundlagenträger enthält, der aus einer Öl-in-Wasseremulsion mit einem pH von etwa 7,0 besteht.

29. Zusammensetzung nach einem der Ansprüche 14 bis 28, worin der Träger eine Cremegrundlage, Emulsion, Lotion oder Pufferlösung ist.

30. Zusammensetzung nach einem der Ansprüche 14 bis 29, worin die Zusammensetzung in Form eines therapeutischen Implantats zur subkutanen Anwendung vorliegt.

31. Zusammensetzung nach einem der Ansprüche 14 bis 29, worin die Zusammensetzung zur subkutanen Verabreichung durch Injektion präpariert ist.

**Revendications**

1. L'emploi d'un complexe métallique d'un ion métallique lourd multivalent et d'un ligand organique polyfonctionnel dans une proportion de 1:1 de l'ion métallique au ligand, le ligand étant soit un acide organique soit un acide organique substitué, le complexe ayant une propriéte de dissociation dans l'eau induite par les protons représentée par une courbe de forme sigmoide sur un système de coordonnées cartésiennes du cologarithme de la concentration en ions métalliques en fonction du cologarithme de la concentration en ions hydrogènes, caractérisé en ce que l'emploi sert à la fabrication d'un médicament pour soulager un trouble inflammatoire chez un animal.

2. Un emploi selon la revendication 1, dans lequel le complexe est un mélange aqueux.

3. Un emploi selon la revendication 2, dans lequel le mélange est oléophile.

4. Un emploi selon l'une quelconque des revendications 2 ou 3, dans lequel le mélange est une dispersion huile et eau.

5. Un emploi selon l'une quelconque des revendications 2 à 4, dans lequel le mélange contient en outre un véhicule pour le complexe.

6. Un emploi selon la revendication 5, dans lequel le véhicule comprend un émulsifiant, une cire hydrocarbonée, un alcool polyhydrique et de l'eau.

7. Un emploi selon l'une quelconque des revendications 5 ou 6, dans lequel le véhicule est une base de crème comprenant du monostéarate de glycéryle en quantité d'environ 11% p/p, environ 2% p/p de cire blanche, environ 3% p/p de cérésine, environ 4% p/p de vaseline, environ 6% p/p de glycérine, et environ 74% p/p d'eau.

8. Un emploi selon l'une quelconque des revendications précédentes, dans lequel le complexe est dispersé dans un véhicule pour fournir une composition ayant un pH d'environ 6,5 à environ 9, d'environ 7 à 9, ou supérieur à environ 7.

9. Un emploi selon l'une quelconque des revendications précédentes, dans lequel l'ion métallique est le cuivre, le zinc, le nickel, le chrome, le bismuth, le mercure, l'argent ou le cobalt.

10. Un emploi selon l'une quelconque des revendications précédentes, dans lequel l'acide organique substitué est hydroxy-polycarboxylique, amino-polycarboxylique, sulfhydro-polycarboxylique ou phosphinol-polycarboxylique.

11. Un emploi selon l'une quelconque des revendications 1 à 8, dans lequel le complex métallique est un chélate dimétallique alcalin et monométallique lourd d'un acide alpha-hydroxy-polycarboxylique.

12. Un emploi selon la revendication 11, dans lequel le complexe est un citrate dimétallique alcalin monocuivrique (II).

13. Un emploi selon la revendication 12, dans lequel le complexe est le citrate disodique monocuivrique (II).

14. Une composition contenant un complexe monométallique d'un ion métallique lourd multivalent et d'un ligand organique polyfonctionnel dans une proportion de 1:1 de l'ion métallique au ligand, le ligand étant soit un acide organique soit un acide organique substitué, le complexe ayant une propriété de dissociation dans l'eau induite par les protons représentée par une courbe de forme sigmoide sur un système de coordonnées cartésiennes du cologarithme de la concentration en ions métalliques en fonction du cologarithme de la concentration en ions hydrogènes, caractérisée en ce que la composition a un pH qui ne dissociera pas le complexe de façon néfaste, et en ce que la composition est dispersée dans un véhicule soit pour une application pharmaceutique d'anti-inflammatoire local soit pour une administration sous-cutanée.

15. Une composition selon la revendication 14, dans laquelle une composition a un pH supérieur à environ 7.

16. Une composition selon l'une quelconque des revendications 14 ou 15, dans laquelle le complexe est dispersé dans un véhicule ayant un pH compris dans l'intervalle physiologique d'environ 7 à environ 9.

17. Une composition selon l'une quelconque des revendications 14 à 16, dans laquelle l'ion métallique est le cuivre, le zinc, le nickel, le chrome, le bismuth, le mercure, l'argent ou le cobalt.

18. Une composition selon l'une quelconque des revendications 14 à 17, dans laquelle l'acide organique substitué est hydroxy-polycarboxylique, amino-polycarboxylique, sulfhydro-polycarboxylique ou phosphinol-polycarboxylique.

19. Une composition selon l'une quelconque des revendications 14 à 16, dans laquelle le complexe métallique est un chélate dimétallique alcalin monométallique lourd d'un acide alpha-hydroxy-polycarboxylique.

20. Une composition selon l'une quelconque des revendications 14 à 16, dans laquelle le complexe est un citrate dimétallique alcalin monocuivrique (II).

21. Une composition selon l'une quelconque des revendications 14 à 20, dans laquelle le complexe est un mélange aqueux.

22. Une composition selon la revendication 21, dans laquelle le mélange est oléophile.

23. Une composition selon l'une quelconque des revendications 14 à 22, dans laquelle le véhicule comprend un émulsifiant, une cire hydrocarbonée, un alcool polyhydrique et de l'eau.

24. Une composition selon la revendication 23, dans laquelle les véhicule est une base de crème comprenant du monostéarate de glycéryle en tant qu'émulsifiant.

25. Une composition selon l'une quelconque des revendications 14 à 24, dans laquelle le véhicule comprend du monostéarate de glycéryle, de la cire blanche, de la cérésine, de la vaseline, de la glycérine et de l'eau.

26. Une composition selon l'une quelconque des revendications 14 à 16, dans laquelle le complexe métallique est renfermé dans une dispersion d'huile et d'eau avec un agent émulsifiant choisi parmi les agents anioniques et non-anioniques en fournissant dans la phase aqueuse un citrate dimétallique alcalin monocuivrique (II), en quantité efficace anti-inflammatoire, à un pH d'environ 7 à environ 9.

27. Une composition selon la revendication 26, dans laquelle le citrate dimétallique alcalin monocuivrique (II) représente environ 5% à environ 15% p/p de la composition.

28. Une composition selon l'une quelconque des revendications 26 ou 27, dans laquelle la composition comprend du citrate disodique monocuivrique (II) en quantité d'environ 10% p/p dans un véhicule à base de crème hydrodispersible, comprenant une émulsion huile dans l'eau ayant un pH d'environ 7,0.

29. Une composition selon l'une quelconque des revendications 14 à 28, dans laquelle le véhicule est soit une base de crème, une émulsion, une lotion soit une solution tamponnée.

30. Une composition selon l'une quelconque des revendications 14 à 29, dans laquelle la composition est sous forme d'un implant thérapeutique pour administration sous-cutanée.

31. Une composition selon l'une quelconque des revendications 14 à 29, dans laquelle la composition est préparée pour l'administration sous-cutanée par injection.